# FASCICULE DE BREVET EUROPEEN

(11) **EP 3 896 632 B1**
(45) Date de publication et mention de la délivrance du brevet: **13.03.2024**
(21) Numéro de dépôt: 20170215.6
(22) Date de dépôt: 17.04.2020
(51) Int. Cl.: G06Q 10/10, G16H 50/00, G16H 50/80

(54) **PROCÉDÉ ET SYSTÈME DE GESTION D'UNE ÉPIDÉMIE SANITAIRE SUR UN TERRITOIRE**
MANAGEMENTVERFAHREN UND -SYSTEM EINER EPIDEMIE IN EINEM GEBIET
METHOD AND SYSTEM FOR MANAGING A HEALTH EPIDEMIC IN A TERRITORY

(43) Date de publication de la demande: 20.10.2021
(73) Titulaire: Bull SAS, 78340 Les Clayes-sous-Bois (FR)
(72) Inventeur: BENRACHI, Samia, 13127 VITROLLES (FR); WENSINK, Emmanuel, 3006 BERN (CH); LORMETEAU, Simon, 35760 SAINT GREGOIRE (FR); KHALLEF, Walid, 75011 PARIS (FR)
(74) Mandataire: Argyma

(56) Documents cités:
- KR-A- 20170 021 692
- US-A1- 2007 106 775
- US-A1- 2010 238 023
- US-A1- 2012 215 455
- US-A1- 2017 264 507
- US-A1- 2017 352 119

## Description

### DOMAINE TECHNIQUE

La présente invention concerne le domaine de la gestion d'une épidémie sanitaire sur un territoire donné, en particulier, suite à une contamination d'une pluralité d'individus d'une population par un ou plusieurs virus.

Lorsqu'un individu est porteur d'un virus et présente des symptômes, il requiert des soins pour améliorer sa santé. Par la suite, un tel individu est désigné « individu malade ». Lorsqu'un individu ne présente pas de symptôme (asymptomatique), celui-ci peut être porteur du virus et susceptible de le transmettre à d'autres individus. On parle alors d'un « individu porteur ». Un individu porteur est susceptible de contaminer un « individu sain », c'est-à-dire, un individu qui ne présente pas de symptôme et qui n'est pas porteur du virus. La transmission du virus est réalisée principalement par contact entre un individu porteur et un individu sain.

Lorsqu'un individu porteur est identifié, par exemple lors d'un test viral, il est souhaité de prévenir les personnes qui ont été en contact avec l'individu porteur afin qu'elles puissent être testées. De manière incidente, on connaît par les documents US 2012/238023, KR 2017 0021692 A des procédés de gestion d'une épidémie sanitaire.

Un des objectifs de la présente invention est de déterminer les porteurs potentiels afin de mieux contrôler la propagation du virus.

### PRESENTATION DE L'INVENTION

L'invention concerne un procédé, selon la revendication 1, pour la gestion d'une épidémie sanitaire due à au moins un virus sur un territoire prédéterminé au moyen d'une plateforme de gestion informatique, le territoire comportant une pluralité de porteurs identifiés, chaque porteur identifié étant équipé d'au moins un équipement de télécommunication associé à un identifiant, le procédé comprenant :
- une étape de fourniture d'une base de proximité associant un identifiant à une pluralité de positions géographiques horodatées,
- une étape de détermination, pour au moins un porteur identifié d'au moins un identifiant primaire,
- une étape de recherche dans la base de proximité de l'ensemble des positions géographiques horodatées pour ledit identifiant primaire sur une période de temps de contagion prédéterminée,
- une étape de détermination, dans la base de proximité, d'identifiants secondaires, ayant des positions géographiques horodatées secondaires qui sont communes avec les positions géographiques horodatées primaires, de manière à déterminer une base de porteurs potentiels, et
- une étape de contact des individus associés aux identifiants secondaires de la base de porteurs potentiels.

De manière avantageuse, le présent procédé de gestion permet de former une base de porteurs potentiels tout en respectant les données personnelles des individus. Les individus porteurs sont avantageusement contactés tout en restant anonymes,

Selon l'invention, l'étape de détermination comporte :
- une sous-étape de classification des positions géographique primaires de chaque individu primaire selon des plages temporelles de durée prédéterminée,
- une sous-étape de détermination, pour chaque plage temporelle, d'une région de contagion dans laquelle sont incluses lesdites positions géographiques primaires,
- une sous-étape de détermination, dans la base de proximité, d'identifiants secondaires, ayant des positions géographiques horodatées secondaires qui appartiennent, d'une part, temporellement à ladite plage temporelle et, d'autre part, géographiquement à la région de contagion de manière à déterminer une base de porteurs potentiels.

La détermination de régions de contagion permet de faciliter la sous-étape de détermination à partir de la base de proximité pouvant comporter plusieurs millions d'individus.

De préférence, la région de contagion approxime lesdites positions géographiques primaires par une forme géométrique, de préférence, polygonale. La région de contagion permet de tenir compte des imprécisions de la position géographique.

Selon l'invention, le procédé comprend :
- une sous-étape d'agrégation de régions de contagion obtenues sur une plage temporelle globale, correspondant à plusieurs plages temporelles consécutives de manière à déterminer une région de contagion globale et
- une sous-étape de détermination, dans la base de proximité, d'identifiants secondaires, ayant des positions géographiques horodatées secondaires qui appartiennent, d'une part, temporellement à ladite plage temporelle globale et, d'autre part, géographiquement à la région de contagion globale de manière à déterminer une base de porteurs potentiels .

Selon l'invention, le procédé comprend :
- une sous-étape de détermination de la durée d'apparition des identifiants secondaires dans ladite plage temporelle globale et
- une sous-étape de conservation des identifiants secondaires dont la durée d'apparition est supérieure à un premier seuil de temps prédéterminé.

Plus un individu demeure longtemps dans une région contagieuse, plus son risque de contamination est élevé.

Selon l'invention, le procédé comprend :
- une sous-étape de détermination d'au moins une zone de superposition de régions de contagion obtenues sur une plage temporelle globale, correspondant à plusieurs plages temporelles consécutives
- une sous-étape de détermination, dans la base de proximité, d'identifiants secondaires, ayant des positions géographiques horodatées secondaires qui appartiennent, d'une part, temporellement à ladite plage temporelle globale et, d'autre part, géographiquement à la zone de superposition
- une sous-étape de détermination de la durée d'apparition des identifiants secondaires dans ladite plage temporelle globale et
- une sous-étape de conservation des identifiants secondaires dont la durée d'apparition est supérieure à un deuxième seuil de temps prédéterminé.

De manière préférée, le premier seuil de temps est plus grand que le deuxième seuil de temps. Ainsi, lorsqu'une région de contagion est plus contagieuse qu'une autre, on peut considérer un individu comme porteur potentiel même si sa durée de présence est plus faible.

Selon un aspect préféré, la région de contagion est polygonale.

De préférence, la période de temps de contagion est comprise entre 3 jours et 21 jours. De préférence encore, les accès à la base de proximité sont réalisés de manière cryptée.

Selon un aspect de l'invention, une position géographique horodatée secondaire est considérée comme commune avec une position géographique horodatée primaire lorsque l'écart géographique est inférieur à 10 m, de préférence, 5 m.

De préférence, une position géographique horodatée secondaire est considérée comme commune avec une position géographique horodatée primaire lorsque les positions géographiques sont communes sur une durée supérieure à 1 minute.

Il est également présenté, sans faire partie de l'invention, un procédé de gestion d'une épidémie sanitaire due à au moins un virus sur un territoire prédéterminé au moyen d'une plateforme de gestion informatique, le territoire comportant une pluralité d'individus, chaque individu étant équipé d'au moins un équipement de télécommunication comprenant une application informatique pour collecter les identifiants horodatés d'équipements d'autres individus situés à proximité, le procédé comprenant :
- une étape d'obtention, pour au moins un individu porteur identifié, des identifiants horodatés collectés par l'application informatique dudit individu porteur identifié sur une période de temps de contagion déterminée, et
- une étape de contact des individus associés aux identifiants horodatés.

Selon un aspect ne faisant pas partie de l'invention, l'application informatique de chaque équipement de télécommunication étant configurée pour communiquer avec la plateforme de gestion informatique, chaque application informatique ayant au moins un état choisi parmi les états suivants « sain », « suspect », « malade » ou « guéri », lors de l'étape de contact, la plateforme de gestion informatique est configurée pour contacter les applications associées auxdits identifiants horodatés et pour passer celles qui sont dans un état « sain » à un état « suspect ».

L'invention concerne également un programme informatique pour la mise en oeuvre du procédé tel que présenté précédemment et un support de mémoire informatique comprenant ledit programme informatique.

### PRESENTATION DES FIGURES

L'invention sera mieux comprise à la lecture de la description qui va suivre, donnée à titre d'exemple, et se référant aux figures suivantes, données à titre d'exemples non limitatifs, dans lesquelles des références identiques sont données à des objets semblables.
La figure 1 est une représentation schématique d'étapes pour la détermination d'une base de porteurs potentiels.
La figure 2A est une représentation schématique de détermination d'une base de porteurs potentiels par une région de contagion.
La figure 2B est une représentation schématique de détermination d'une base de porteurs potentiels par une région de contagion globale.
La figure 2C est une représentation schématique de détermination d'une base de porteurs potentiels par une région de contagion globale comportant une pluralité de zones de superposition.
La figure 3 est une représentation schématique de détermination de données statistiques et de données géographiques à partir de la base de porteurs potentiels.
La figure 4 est une représentation schématique d'une forme de réalisation d'une architecture d'un système de gestion d'une épidémie sanitaire.
La figure 5 (ne faisant pas partie de l'invention) est une représentation schématique d'étapes pour la détermination d'une base de porteurs potentiels au moyen d'une application de collecte d'identifiants horodatés.
La figure 6 est une représentation schématique du changement d'états d'applications par la plateforme de gestion informatique suite à différents déclencheurs.

Il faut noter que les figures exposent l'invention de manière détaillée pour mettre en oeuvre l'invention, lesdites figures pouvant bien entendu servir à mieux définir l'invention le cas échéant.

### DESCRIPTION DETAILLEE DE L'INVENTION

L'invention concerne un procédé de gestion d'une épidémie sanitaire due à au moins un virus sur un territoire prédéterminé, en particulier, une région, une nation ou un continent.

Au cours d'une épidémie virale, le territoire comporte une pluralité d'individus malades M, une pluralité d'individus porteurs P et une pluralité d'individus sains S. Comme indiqué précédemment, parmi les individus asymptomatiques, il existe des individus porteurs P et des individus sains S.

La présente invention vise à déterminer et informer les porteurs potentiels P afin de mieux contrôler la propagation d'une épidémie.

L'invention trouve une application pour les individus qui possèdent au moins un équipement de télécommunication, de préférence connecté au réseau téléphonique, par exemple, un téléphone mobile, une tablette, etc. Par la suite, on considère que chaque individu M, P, S est équipé d'au moins un équipement de télécommunication associé à un identifiant NUM.

De manière préférée, l'identifiant NUM est un numéro IMSI pour « International Mobile Subscriber Identity » ou MSISDN pour « Mobile Station ISDN Number ». Par souci de concision, l'identifiant d'un équipement d'un équipement mobile NUM est désigné par la suite l'identifiant NUM.

De manière connue, pour chaque numéro de téléphone, il existe un unique identifiant NUM. Comme cela sera présenté par la suite, lorsqu'un individu est porteur P ou malade M, celui-ci peut accepter de fournir son identifiant NUM afin de détecter des porteurs potentiels PP.

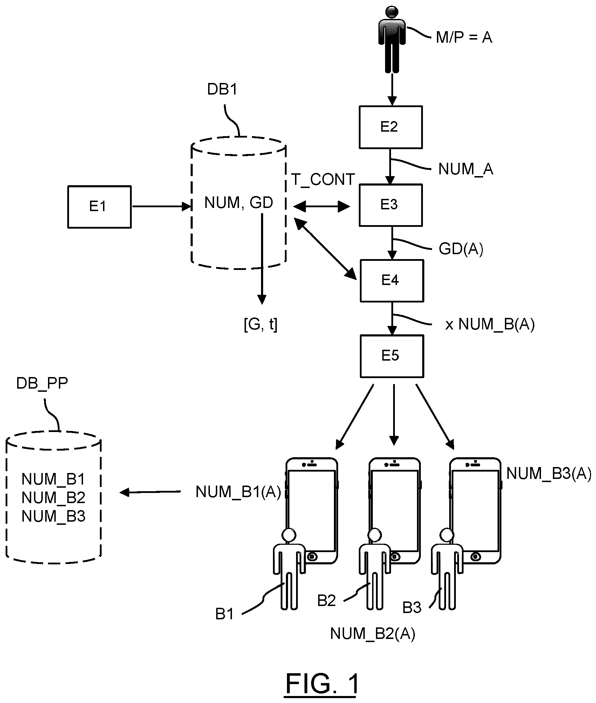

Selon l'invention, en référence à la figure 1, le procédé comporte une étape de fourniture E1 d'une base de proximité DB1 associant un identifiant NUM à une pluralité de positions géographiques horodatées GD. Une telle base de proximité DB1 est connue de l'homme du métier. Dans cet exemple, la base de proximité B1 utilise un système e « LBS » pour « Location Base Services » comprenant des données obtenues à partir des infrastructures du réseau téléphonique et de données. De manière avantageuse, les identifiants NUM sont anonymisés et l'accès à cette donnée ne compromet pas les données personnelles des utilisateurs (nom, prénom, sexe, etc.). Il va de soi que la base de proximité B1 pourrait utiliser des données de sources diverses, par exemple, de plateformes réseaux ou d'applications partageant des localisations.

De manière préférée, la base de proximité DB1 associe un numéro de téléphone à chaque identifiant NUM. Il va de soi que le numéro de téléphone pourrait remplir la fonction d'identifiant NUM.

Une position géographique horodatée GD correspond à un couple de données comportant, d'une part, une position géographique G et, d'autre part, un horodatage t. A titre d'exemple, pour un identifiant NUM, il peut être déterminé que l'équipement mobile était jeudi 26 mars à 13h à une première adresse (par exemple celle d'un restaurant) et vendredi 27 mars à 22h à une deuxième adresse (par exemple celle d'une habitation résidentielle). De manière connue, l'horodatage t est obtenu directement par l'infrastructure du réseau téléphonique et de données. La position géographique G peut être obtenue par localisation GPS, triangulation ou analogue.

Autrement dit, la base de proximité DB1 comporte un historique des positions géographiques horodatées pour un identifiant NUM et, par voie de conséquence, de celles de son propriétaire qui demeure anonyme.

Le procédé comporte une étape de détermination E2, pour au moins un individu porteur P, d'au moins un identifiant d'équipement mobile primaire NUM_A. On entend par individu primaire A, un individu qui a reçu un test viral et dont on est certain qu'il est porteur du virus avec ou sans symptômes (M ou P). Par souci de concision et de clarté, il va être supposé que l'individu primaire A ne possède qu'un téléphone mobile associé à un unique identifiant primaire NUM_A .

Dans cet exemple, lors d'un test viral, l'individu primaire A donne son accord explicite pour accéder à son numéro de téléphone afin de permettre de localiser les porteurs potentiels qu'il est susceptible d'avoir contaminé. Lorsque le test viral est positif, il est déterminé l'identifiant primaire NUM_A.

Le procédé comporte une étape de recherche E3 dans la base de proximité DB1 de l'ensemble des positions géographiques horodatées primaires GD(A) pour ledit identifiant primaire NUM_A sur une période de temps de contagion déterminée T_CONT. A titre d'exemple, la période de contagion T_CONT est comprise entre 3 jours et 21 jours.

Le procédé comporte une étape de détermination E4, dans la base de proximité DB1, d'identifiants secondaires NUM_B(A), ayant des positions géographiques horodatées secondaires GD(B) qui sont communes avec les positions géographiques horodatées primaires GD(A) de manière à déterminer une base de porteurs potentiels DB_PP. Autrement dit, par analyse de la base de proximité DB1 sur une période contagion déterminée T_CONT, on détermine les identifiants secondaires NUM_B(A) ayant été en contact avec l'individu primaire A, c'est-à-dire, des porteurs potentiels.

L'obtention d'une base de porteurs potentiels DB_PP est primordiale au début d'une épidémie étant donné qu'elle permet de circonscrire les foyers de contamination (« cluster » en langue anglaise).

Selon l'invention, le procédé comporte une étape de contact E5 des individus secondaires B associés aux identifiants secondaires NUM-B(A). L'étape de contact peut avantageusement être réalisée sans compromettre leurs données personnelles étant donné que seuls les identifiants secondaires NUM-B(A) sont connus, les individus secondaires B restant anonymes.

L'étape de contact peut se présenter sous la forme d'un appel téléphonique ou d'un message texte (SMS et analogues) afin d'informer les individus secondaires B qu'ils sont probablement porteurs, qu'ils sont incités à se confiner ou qu'ils sont incité à réaliser un test viral.

De manière avantageuse, on peut réaliser une campagne de dépistage de manière prédictive pour limiter le risque de progression de la contamination.

Il a été déterminé des individus secondaires B à partir d'un unique individu primaire A mais il va de soi que ces étapes peuvent être répétés pour chaque individu primaire A afin d'augmenter le nombre d'individus de la base de porteurs potentiels DB_PP.

De manière à améliorer la pertinence de la base de porteurs potentiels DB_PP. Le procédé comporte, lors de l'étape de détermination E4, une sous-étape de filtrage des positions géographiques horodatées secondaires GD(B) qui sont communes avec les positions géographiques horodatées primaires GD(A).

De préférence, une position géographique horodatée secondaire GD(B) est considérée comme commune avec une position géographique horodatée primaire GD(A) lorsque l'écart géographique est inférieur à 10 m, de préférence, inférieure à 5 m. Un filtrage par distance permet d'augmenter la probabilité de ne sélectionner que des individus qui sont susceptibles d'avoir été contaminés. De préférence encore, une position géographique horodatée secondaire GD(B) est considérée comme commune avec une position géographique horodatée primaire GD(A) lorsque les positions géographiques sont communes sur une durée supérieure à 1 minutes. Un filtrage par durée de contact permet d'augmenter la probabilité de ne sélectionner que des individus qui sont susceptibles d'avoir été contaminés.

Pour déterminer, dans la base de proximité D1B, les identifiants secondaires NUM_B(A), ayant des positions géographiques horodatées secondaires GD(B) qui sont communes avec les positions géographiques horodatées primaires GD(A), il est proposé de déterminer une région de contagion RC pour chaque individu primaire pour une plage temporelle déterminée. Dans cet exemple de mise en oeuvre, la distance entre individus n'est pas calculée en tant que tel.

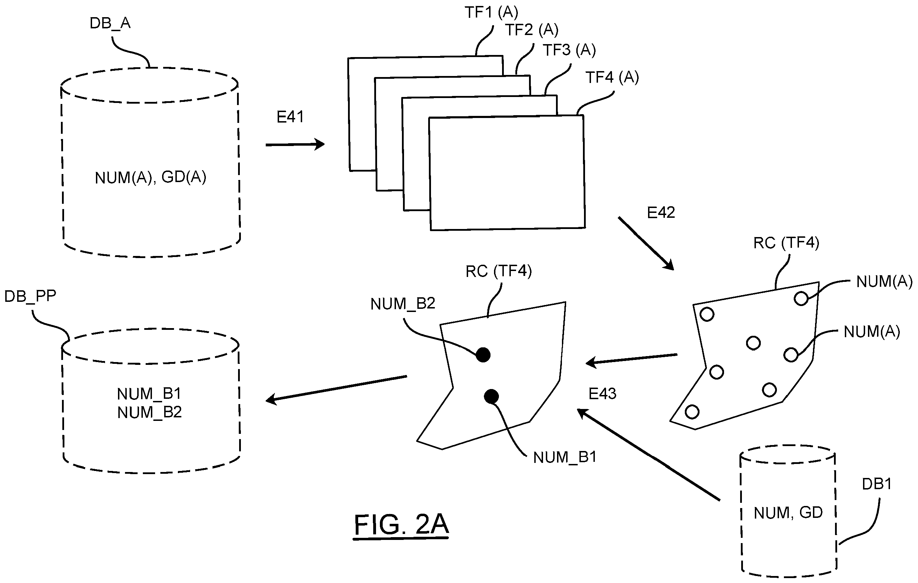

En référence à la figure 2A, le procédé comporte :
- une sous-étape de classification E41 des positions géographique primaires G(A) de chaque individu primaire A selon des plages temporelles TF de durée prédéterminée,
- une sous-étape de détermination E42, pour chaque plage temporelle TF, d'une région de contagion RC dans laquelle sont incluses lesdites positions géographiques primaires G(A),
- une sous-étape de détermination E43, dans la base de proximité DB1, d'identifiants secondaires NUM_B(A), ayant des positions géographiques horodatées secondaires GD(B) qui appartiennent, d'une part, temporellement à ladite plage temporelle TF et, d'autre part, géographiquement à la région de contagion RC de manière à déterminer une base de porteurs potentiels DB_PP.

De manière analogue à précédemment, les individus associés aux identifiants secondaires NUM_B(A) de la base de porteurs potentiels DB_PP sont contactés.

A titre d'illustration, en référence à la figure 2A, il a été préalablement déterminé, pour un individu primaire A, malade M ou porteur P, son identifiant primaire NUM_A. Suite à une étape de recherche dans la base de proximité DB1, on dispose de l'ensemble des positions géographiques horodatées primaires GD(A) pour ledit identifiant primaire NUM_A sur la période de temps de contagion déterminée T_CONT propre à chaque individu primaire. Chaque position géographiques horodatée primaire GD comprend une position géographique primaire associée à un horodatage. On dispose ainsi d'une base de proximité primaire DB_A.

Toujours en référence à la figure 2A, les positions géographiques primaires sont classifiées selon des plages temporelle TF1, TF2, TF3, TF4 de durée prédéterminée. Dans cet exemple, la durée prédéterminée est comprise entre 5 minutes et 20 minutes, de préférence, 10 minutes. La durée d'une plage temporelle TF1, TF2, TF3, TF4 est choisie de manière à correspondre à la durée pour laquelle une zone demeure contagieuse. Le but est identifier la zone géographique qui est, en tant que tel, contagieuse même en l'absence d'un individu primaire. Dans cet exemple, de la figure 2a, quatre plages temporelles TF1, TF2, TF3, TF4 sont déterminées.

Pour chaque plage temporelle TF1, TF2, TF3, TF4 propre à chaque identifiant primaire NUM_A, on détermine une région de contagion RC dans laquelle sont incluses lesdites positions géographiques primaires G(A) datées pendant ladite plage temporelle TF1, TF2, TF3, TF4. De préférence, la région de contagion RC approxime lesdites positions géographiques primaires G(A) par une forme géométrique, de préférence, polygonale. De préférence, le périmètre de la forme géométrique approxime les positions géographiques les plus éloignées dans la plage temporelle tout en tenant compte des imprécisions de la mesure de la position géographique.

La dimension et/ou la forme de la région de contagion RC est déterminée de manière dynamique en fonction de la répartition des positions géographiques primaires dans une plage temporelle déterminée TF. En référence à la figure 2a, la région de contagion RC est de forme polygonale et englobe 7 positions géographiques primaires G(A) de l'identifiant primaire NUM_A.

On peut ainsi déterminer, dans la base de proximité DB1, les identifiants secondaires NUM_B1, NUM_B2, ayant des positions géographiques horodatées secondaires qui appartiennent, d'une part, temporellement à ladite plage temporelle TF4 et, d'autre part, géographiquement à la région de contagion RC de manière à déterminer une base de porteurs potentiels DB_PP afin de les contacter. Une détermination par région de contagion est moins consommatrice de ressources informatiques qu'un calcul de distances entre individus.

Bien qu'en respectant une distanciation sociale, des individus sont considérés comme porteurs potentiels pour avoir été présents au mauvais endroit (région de contagion RC) au mauvais moment (plage temporelle prédéterminée TF4).

Selon un aspect préféré, dans la base de porteurs potentiels DB_PP, chaque identifiant NUM est associé à un score qui est fonction du nombre d'occurrences dudit identifiant NUM dans une plage temporelle TF4 pour une région de contagion RC déterminée. Plus le score est élevé, plus la probabilité que l'individu soit porteur est élevée. Un tel individu est contacté en priorité.

Comme expliqué précédemment, il est déterminé dans la base de proximité DB1, les identifiants secondaires NUM_B1, NUM_B2, ayant des positions géographiques horodatées secondaires qui appartiennent à ladite plage temporelle TF4. Néanmoins, une telle étape peut être consommatrice en ressources informatiques étant donné que la base de proximité DB1 peut comprendre une pluralité de millions d'étapes

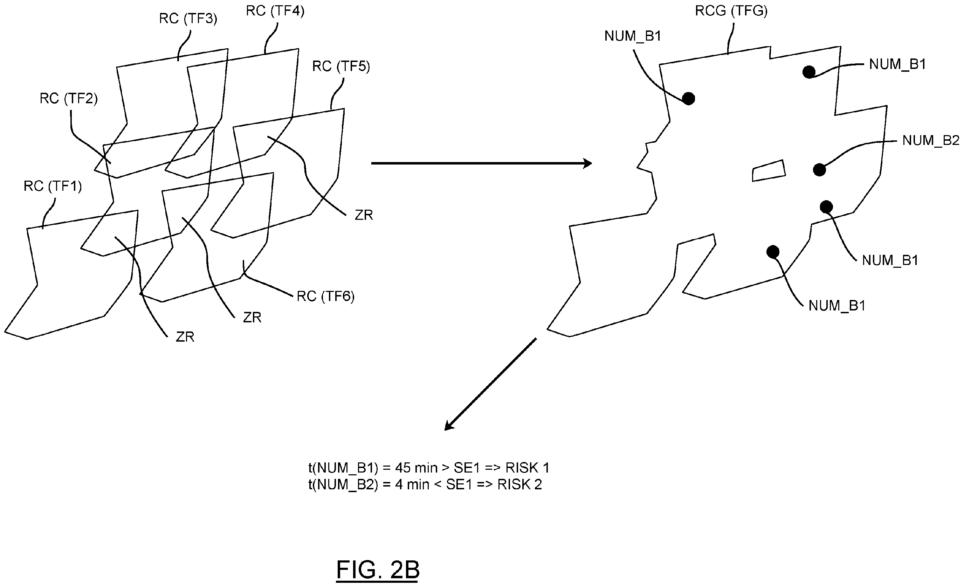

En référence à la figure 2B, le procédé comporte une sous-étape d'agrégation de régions de contagion RC obtenues sur une plage temporelle globale TFG, correspondant à plusieurs plages temporelles consécutives TF de manière à déterminer une région de contagion globale RCG. Dans cet exemple, la plage temporelle globale TFG correspond à six plages temporelles TF et dure ainsi 60 minutes mais il va de soi que le nombre de plages pourrait être différent. Dans l'exemple de la figure 2B, il est représenté des régions de contagion RC qui sont de formes identiques pour chaque plage temporelle TF mais il va de soi qu'elles pourraient être de formes différentes en fonction des déplacements de l'identifiant primaire A.

De manière analogue à précédemment, le procédé comporte une sous-étape de détermination, dans la base de proximité DB1, d'identifiants secondaires NUM_B(RC), ayant des positions géographiques horodatées secondaires GD(B) qui appartiennent, d'une part, temporellement à ladite plage temporelle globale TFG et, d'autre part, géographiquement à la région de contagion globale RCG de manière à déterminer une base de porteurs potentiels DB_PP. Un tel procédé est avantageux étant donné que la sous-étape de détermination n'est mise en oeuvre que pour chaque plage temporelle globale TFG et non pour chaque plage temporelle TF, ce qui réduit les ressources informatiques étant donné que la base de proximité DB1 comporte des millions d'identifiants qui sont associés chacun à de nombreuses positions géographiques horodatées secondaires GD(B).

De manière préférée, les identifiants secondaires NUM_B(RC) sont filtrés de manière à sélectionner ceux dont le risque est le plus élevé (RISK1) tout en mettant de côté ceux dont le risque est plus faible (RISK2).

A cet effet, comme illustré à la figure 2B, le procédé comporte :
- une sous-étape de détermination de la durée d'apparition des identifiants secondaires NUM_B(RC) dans ladite plage temporelle globale TFG et
- une étape de conservation des identifiants secondaires NUM_B(RC) dont la durée d'apparition est supérieure à un premier seuil de temps prédéterminé SE1.

Autrement dit, seuls les identifiants secondaires NUM_B(RC) présents pendant une longue période sont considérés comme risqués (RISK1). Dans cet exemple, le premier seuil de temps prédéterminé SE1 est choisi égal à 30 minutes. En référence à la figure 1, Un identifiant secondaire NUM_B1 dont la durée d'apparition est de 45 minutes est considéré comme un risque élevé (RISK1) tandis qu'un identifiant secondaire NUM_B2 dont la durée d'apparition est de 4 minutes est considéré comme un risque faible (RISK2).

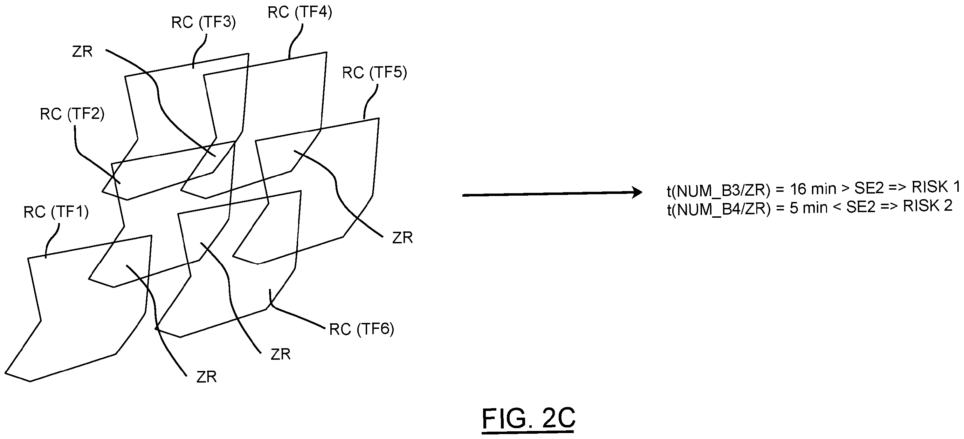

Afin d'améliorer encore le filtrage, en référence à la figure 2C, le procédé de gestion comprend une sous-étape détermination d'au moins une zone de superposition de régions de contagion RC obtenues sur une plage temporelle globale TFG, correspondant à plusieurs plages temporelles consécutives TF. Par définition, une zone de superposition ZR est plus susceptible d'héberger le virus étant donné qu'elle a été plusieurs fois visitées sur une période courte (plage temporelle globale TFG).

Le procédé comporte :
- une sous-étape de détermination, dans la base de proximité DB1, d'identifiants secondaires NUM_B(RC), ayant des positions géographiques horodatées secondaires GD(B) qui appartiennent, d'une part, temporellement à ladite plage temporelle globale TFG et, d'autre part, géographiquement à la zone de superposition ZR
- une sous-étape de détermination de la durée d'apparition des identifiants secondaires NUM_B(RC) dans ladite plage temporelle globale TFG et
- une étape de conservation des identifiants secondaires NUM_B(RC) dont la durée d'apparition est supérieure à un deuxième seuil de temps prédéterminé SE2.

De manière avantageuse, la durée d'apparition d'un identifiant secondaire NUM_B(RC) est comparée à un deuxième seuil de temps prédéterminé SE2 qui est plus faible de que le premier seuil SE1 de manière à déclarer un risque élevé (RISK1) de manière plus rapide. A titre d'exemple, le deuxième seuil de temps prédéterminé SE2 est de 15 minutes.

Ainsi, si un individu demeure peu de temps dans une zone très contagieuse, il est déclaré avec un risque élevé.

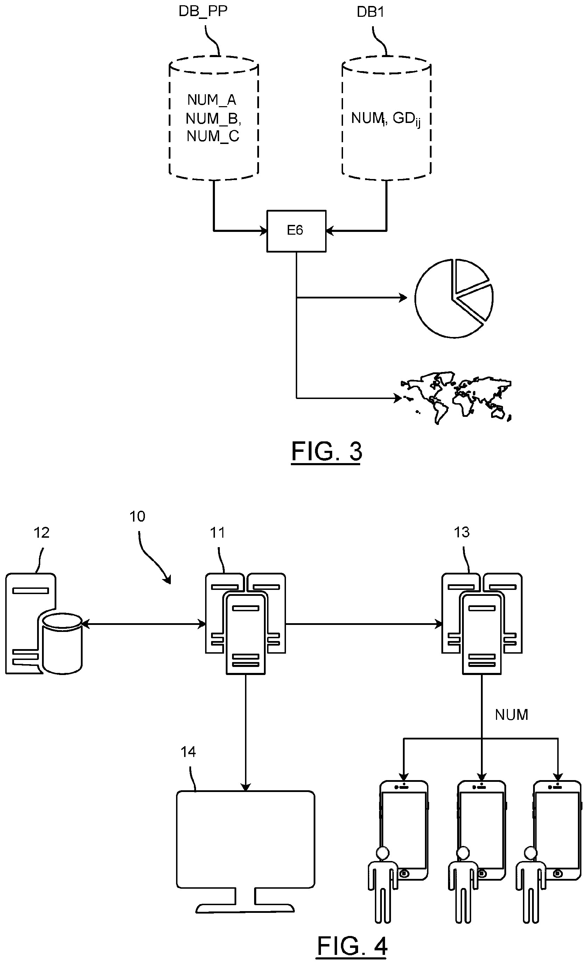

Une fois obtenue les identifiants secondaires, on dispose d'une base de porteurs potentiels DB_PP qui peut être exploitée de différentes manières. En référence à la figure 3, on peut réaliser une étape de localisation E6 des porteurs potentiels en croisant la base de porteurs potentiels DB_PP et la base de proximité DB, en particulier, en lisant la dernière position géographique des identifiants NUM_A, NUM_B, NUM_C, etc. Suite à cette étape de localisation, on peut réaliser des statistiques d'individus porteurs par localisation et ainsi identifier des potentiels foyers, déterminer le nombre de porteurs potentiels, obtenir une cartographie représentant la densité des porteurs, etc.

Il a été présenté précédemment un identifiant NUM relatif à identifiant de communication mais il va de soi qu'il pourrait être de nature différente par exemple, un identifiant d'une application, d'un équipement, un identifiant Bluetooth, IMSI, adresse MAC, IMEI ou autre.

Les aspects liés à la sécurité des données, leur cryptage et anonymisation ont été pris en compte à chaque étape du procédé et sont connus de l'homme du métier. Aussi, par souci de concision ou de clarté, ils ne seront pas détaillés de nouveau.

En référence à la figure 4, il est représenté une plateforme de gestion informatique 10 pour la mise en oeuvre du procédé de gestion selon l'invention.

La plateforme de gestion informatique 10 comporte au moins un calculateur de traitement 11 qui, comporte de préférence un ou plusieurs serveurs, au moins un dispositif de gestion de bases de données 12 dans lequel est hébergée la base de proximité DB1 et au moins un serveur de communication téléphonique 13 configuré pour contacter téléphoniquement les individus associés aux identifiants NUM de la base de porteurs DB_PP. Le plateforme de gestion informatique 10 comporte en outre un terminal informatique (PC, tablette, téléphone) permettant l'affichage de potentiels foyers, du nombre de porteurs potentiels, d'une cartographie représentant la densité des porteurs, etc.

Dans l'exemple de mise en oeuvre précédemment présenté, l'individu a donné spécifiquement son accord pour accéder à ses sonnées personnelles. Néanmoins, il va de soi que l'invention pourrait également être mise en oeuvre sans accord de l'individu dans des juridictions ayant un cadre réglementaire autorisant plus largement l'accès aux données personnelles.

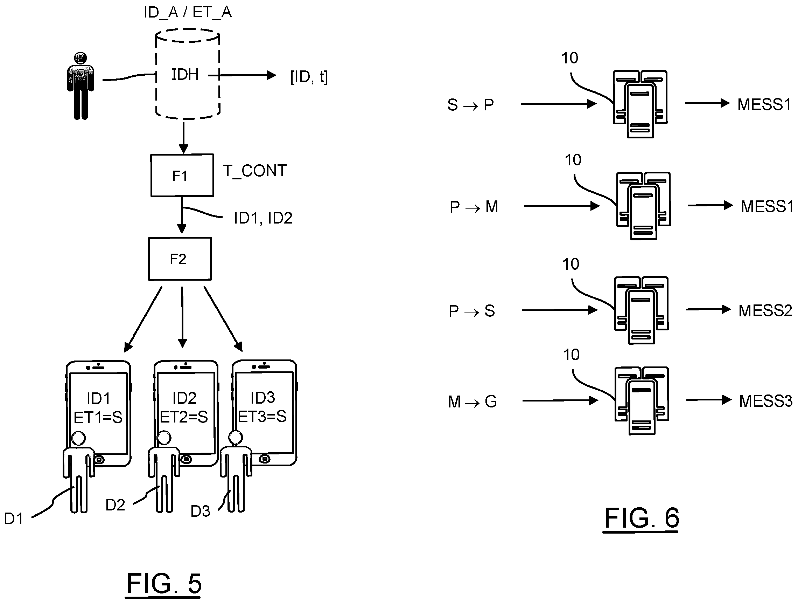

En référence à la figure 5, il est également présenté un procédé de gestion d'une épidémie sanitaire due à au moins un virus sur un territoire prédéterminé au moyen d'une plateforme de gestion informatique 10, le territoire comportant une pluralité d'individus S, M, P, chaque individu étant équipé d'au moins un équipement de télécommunication comprenant une application informatique pour collecter les identifiants horodatés IDH d'équipements d'autres individus situés à proximité.

Dans cet exemple, la plateforme de gestion informatique 10 est la même que celle présentée précédemment mais il va de soi que les deux plateformes pourraient être différentes. L'utilisation des deux procédés au sein d'une même plateforme de gestion informatique 10 est avantageuse car elle permet de gérer la propagation de manière globale et de disposer de synergies, en particulier, pour gérer les messages aux porteurs potentiels. Les deux procédés peuvent être mis en oeuvre de manière indépendante ou dépendante.

Une application informatique pour collecter les identifiants horodatés IDH d'équipements d'autres individus situés à proximité est connue en soi de l'homme du métier et ne sera pas présentée en détails. L'application met par exemple en oeuvre une technologie basée sur le Bluetooth ou sur l'internet des objets (loT). De préférence, les identifiants horodatés sont ceux obtenus pendant une période d'au moins 10 minutes qui ont été détectés à moins de 5 m.

L'application informatique permet de communiquer avec la plateforme de gestion informatique 10 par le réseau de communication comme cela sera présenté par la suite, à titre d'exemple, pour plusieurs scénarii prédéterminés.

Selon l'invention, le procédé comprend une étape d'obtention F1, pour au moins un individu porteur identifié P, des identifiants horodatés IDH collectés par l'application informatique dudit individu porteur identifié P sur une période de temps de contagion déterminée T_CONT. L'étape d'obtention F1 peut être réalisée de différentes manières, par téléchargement des données par la plateforme de gestion informatique 10, par lecture à partir d'un objet connecté, par exemple, tel que décrit dans la demande de brevet FR3076382 du demandeur. L'étape d'obtention F1 est, par exemple, réalisée suite à un test de dépistage positif.

Le procédé comporte en suite une étape de contact F2 des individus associés aux identifiants horodatés IDH. De préférence, chaque identifiant horodaté IDH est associé à un équipement de télécommunication comprenant une application mobile apte à communiquer avec la plateforme de gestion informatique 10. Autrement dit, la plateforme de gestion informatique 10 permet de réaliser une intermédiation entre les applications mobiles des différents équipements de télécommunication afin d'échanger des données tout en préservant l'anonymat des utilisateurs.

De manière préférée, chaque application informatique possède un état ET choisi parmi les états suivants « sain S », « suspect P », « malade M » ou « guéri G », lors de l'étape de contact F2, la plateforme de gestion informatique 10 est configurée pour contacter les applications informatique associées auxdits identifiants horodatés IDH et pour modifier celles qui sont dans un état « sain S » à un état « suspect P ». De manière préférée, en référence à la figure 6, une notification MESS1 est envoyée par la plateforme de gestion informatique 10 pour inciter l'utilisateur à procéder à un dépistage du virus.

De manière avantageuse, la plateforme de gestion informatique 10 offre une assistance à chaque événement affectant directement ou indirectement l'utilisateur.

Par exemple, lorsqu'une application mobile modifie son état de « suspect P» à « malade M », cela déclenche un message vers la plateforme de gestion informatique 10 qui émet de manière sécurisée et cryptée, les identifiants horodatés IDH collectés par l'application informatique dudit individu malade M sur une période de temps de contagion déterminée T_CONT. La plateforme de gestion informatique 10 contacte alors les porteurs potentiels afin qu'ils soient incités à réaliser un dépistage. De préférence, le changement d'état ne peut être réalisé que par une autorité compétente de la plateforme de gestion informatique 10 (autorité médicale, etc.) afin d'éviter l'émission de fausses alertes. La plateforme de gestion informatique 10 émet ensuite des notifications MESS1 vers les porteurs potentiels comme présenté précédemment.

De même, lorsqu'une application mobile modifie son état de « suspect P» à « sain S », un message est émis vers la plateforme de gestion informatique 10 qui émet des messages de surveillance MESS2 durant la période d'incubation pour procéder à un second test à la fin de cette période.

Enfin, lorsqu'une application mobile modifie son état de « malade P » à « sain S », la plateforme de gestion informatique 10 maintient l'état « Guéri G » à des fins d'historisation, en cas de récidive des formes plus virulentes et envoi de notification à des fins de suivi pendant la période de pandémie. La plateforme de gestion informatique 10 procédera à une mise à jour des notifications surveillance durant la période d'incubation en le notifiant pour procéder à un second test à la fin de cette période.

## Revendications

1. Procédé de gestion d'une épidémie sanitaire due à au moins un virus sur un territoire prédéterminé au moyen d'une plateforme de gestion informatique (10) comprenant une base de proximité (DB1), le territoire comportant une pluralité de porteurs identifiés (P) du virus, chaque porteur identifié (P) étant équipé d'au moins un équipement de télécommunication associé à un identifiant (NUM), le procédé comprenant :
- une étape de fourniture (E1) de la base de proximité (DB1) associant un identifiant (NUM) à une pluralité de positions géographiques horodatées (GD),
- une étape de détermination (E2), pour au moins un porteur identifié (P) d'au moins un identifiant primaire (NUM_A),
- une étape de recherche (E3) dans la base de proximité (DB1) de l'ensemble des positions géographiques horodatées (GD(A)) pour ledit identifiant primaire (NUM_A) sur une période de temps de contagion prédéterminée (T_CONT),
- une étape de détermination (E4), dans la base de proximité (DB1), d'identifiants secondaires (NUM_B(A)), ayant des positions géographiques horodatées secondaires (GD(B)) qui sont communes avec les positions géographiques horodatées primaires (GD(A)), de manière à déterminer une base de porteurs potentiels (DB_PP) du virus, l'étape de détermination (E4) comprenant :
∘ une sous-étape de classification (E41) des positions géographique primaires (G(A)) de chaque individu primaire (A) selon des plages temporelles (TF) de durée prédéterminée,
∘ une sous-étape de détermination (E42), pour chaque plage temporelle (TF), d'une région de contagion (RC) dans laquelle sont incluses lesdites positions géographiques primaires (G(A)),
∘ une sous-étape de détermination (E43), dans la base de proximité (DB1), d'identifiants secondaires (NUM_B(RC)), ayant des positions géographiques horodatées secondaires (GD(B)) qui appartiennent, d'une part, temporellement à ladite plage temporelle (TF) et, d'autre part, géographiquement à la région de contagion (RC),
∘ une sous-étape d'agrégation de régions de contagion (RC) obtenues sur une plage temporelle globale (TFG), correspondant à plusieurs plages temporelles consécutives (TF),
∘ une sous-étape de détermination, dans la base de proximité (DB1), d'identifiants secondaires (NUM_B(RC)), ayant des positions géographiques horodatées secondaires (GD(B)) qui appartiennent, d'une part, temporellement à ladite plage temporelle globale (TFG) et, d'autre part, géographiquement à la région de contagion globale (RCG),
∘ une sous-étape de détermination de la durée d'apparition des identifiants secondaires (NUM_B(RC)) dans ladite plage temporelle globale (TFG),
∘ une sous-étape de conservation des identifiants secondaires (NUM_B(RC)) dont la durée d'apparition est supérieure à un premier seuil de temps prédéterminé (SE1),
∘ une sous-étape de détermination d'au moins une zone de superposition (ZR) de régions de contagion (RC) obtenues sur une plage temporelle globale (TFG), correspondant à plusieurs plages temporelles consécutives (TF),
∘ une sous-étape de détermination, dans la base de proximité (DB1), d'identifiants secondaires (NUM_B(RC)), ayant des positions géographiques horodatées secondaires (GD(B)) qui appartiennent, d'une part, temporellement à ladite plage temporelle globale (TFG) et, d'autre part, géographiquement à la zone de superposition (ZR),
∘ une sous-étape de détermination de la durée d'apparition des identifiants secondaires (NUM_B(RC)) dans ladite plage temporelle globale (TFG) et
∘ une sous-étape de conservation des identifiants secondaires (NUM_B(RC)) dont la durée d'apparition est supérieure à un deuxième seuil de temps prédéterminé (SE2),
∘ une étape de contact (E5) des individus associés aux identifiants secondaires (NUM_B(A)) de la base de porteurs potentiels (DB_PP).

2. Procédé de gestion selon la revendication 1, dans lequel le premier seuil de temps (SE1) est plus grand que le deuxième seuil de temps (SE2).

3. Programme informatique pour la mise en oeuvre d'un procédé selon l'une des revendications 1 à 2.

4. Support de mémoire informatique comprenant le programme informatique selon la revendication 3.

## Patentansprüche

1. Verwaltungsverfahren einer Gesundheitsepidemie aufgrund mindestens eines Virus in einem vorbestimmten Gebiet mittels einer Informatikverwaltungsplattform (10), umfassend eine Näherungsbasis (DB1), das Gebiet aufweisend eine Vielzahl von identifizierten Trägern (P) des Virus, wobei jeder identifizierte Träger (P) mit mindestens einer Telekommunikationsausrüstung ausgestattet ist, die mit einer Kennung (NUM) verknüpft ist, das Verfahren umfassend:
- einen Schritt des Lieferns (E1) der Näherungsbasis (DB1), die eine Kennung (NUM) mit einer Vielzahl von zeitgestempelten geografischen Positionen (GD) verknüpft,
- einen Schritt des Bestimmens (E2), für mindestens einen identifizierten Träger (P) mindestens einer primären Kennung (NUM_A),
- einen Schritt des Suchens (E3) in der Näherungsbasis (DB1) nach der Gesamtheit der zeitgestempelten geografischen Positionen (GD(A)) für die primäre Kennung (NUM_A) über eine vorbestimmte Ansteckungszeitperiode (T_CONT),
- einen Schritt des Bestimmens (E4), in der Näherungsbasis (DB1), von sekundären Kennungen (NUM_B(A)), die sekundäre zeitgestempelte geografische Positionen (GD(B)) aufweisen, die mit den primären zeitgestempelten geografischen Positionen (GD(A)) gemein sind, sodass eine Basis von potenziellen Trägern (DB_PP) des Virus bestimmt wird, der Schritt des Bestimmens (E4) umfassend:
∘ einen Unterschritt des Klassifizierens (E41) der primären geografischen Positionen (G(A)) jedes primären Individuums (A) gemäß Zeitbereichen (TF) von vorbestimmter Dauer,
∘ einen Unterschritt des Bestimmens (E42), für jeden Zeitbereich (TF), einer Ansteckungsregion (RC), in der die primären geografischen Positionen (G(A)) eingeschlossen sind,
o einen Unterschritt des Bestimmens (E43), in der Annäherungsbasis (DB1), von sekundären Kennungen (NUM_B(RC)), die sekundäre zeitgestempelte geografische Positionen (GD(B)) aufweisen, die einerseits zeitlich zu dem Zeitbereich (TF) und andererseits geografisch zu der Ansteckungsregion (RC) gehören,
o einen Unterschritt des Aggregierens von Ansteckungsregionen (RC), die über einen globalen Zeitbereich (TFG) erhalten werden, der mehreren aufeinanderfolgenden Zeitbereichen (TF) entspricht,
∘ einen Unterschritt des Bestimmens, in der Annäherungsbasis (DB1), von sekundären Kennungen (NUM_B(RC)), die sekundäre zeitgestempelte geografische Positionen (GD(B)) aufweisen, die einerseits zeitlich zu dem globalen Zeitbereich (TFG) und andererseits geografisch zu der globalen Ansteckungsregion (RCG) gehören,
∘ einen Unterschritt des Bestimmens der Dauer des Auftretens der sekundären Kennungen (NUM_B(RC)) in dem globalen Zeitbereich (TFG),
∘ einen Unterschritt des Behaltens von sekundären Kennungen (NUM_B(RC)), deren Dauer des Auftretens größer als ein erster vorbestimmter Zeitschwellenwert (SE1) ist,
∘ einen Unterschritt des Bestimmens mindestens einer Überlagerungszone (ZR) von Ansteckungsregionen (RC), die über einen globalen Zeitbereich (TFG) erhalten werden, der mehreren aufeinanderfolgenden Zeitbereichen (TF) entspricht,
∘ einen Unterschritt des Bestimmens, in der Annäherungsbasis (DB1), von sekundären Kennungen (NUM_B(RC)), die sekundäre zeitgestempelte geografische Positionen (GD(B)) aufweisen, die einerseits zeitlich zu dem globalen Zeitbereich (TFG) und andererseits geografisch zu dem Überlagerungsgebiet (ZR) gehören,
∘ einen Unterschritt des Bestimmens der Dauer des Auftretens der sekundären Kennungen (NUM_B(RC)) in dem globalen Zeitbereich (TFG) und
∘ einen Unterschritt des Behaltens der sekundären Kennungen (NUM_B(RC)), deren Dauer des Auftretens größer als ein zweiter vorbestimmter Zeitschwellenwert (SE2) ist,
∘ einen Schritt des Kontaktierens (E5) von Individuen, die mit sekundären Kennungen (NUM_B(A)) aus der Basis der potenziellen Träger (DB_PP) verknüpft sind.

2. Verwaltungsverfahren nach Anspruch 1, wobei der erste Zeitschwellenwert (SE1) größer als der zweite Zeitschwellenwert (SE2) ist.

3. Computerprogramm für die Ausführung eines Verfahrens nach einem der Ansprüche 1 bis 2.

4. Computerspeicherträger, umfassend das Computerprogramm nach Anspruch 3.

## Claims

1. A method for managing a health epidemic caused by at least one virus in a predetermined territory by means of a computer management platform (10) comprising a proximity base (DB1), the territory comprising a plurality of identified carriers (P) of the virus, each identified carrier (P) being provided with at least one telecommunications apparatus associated with an identifier (NUM), the method comprising:
- a step of providing (E1) the proximity base (DB1) which associates an identifier (NUM) with a plurality of time-stamped geographic positions (GD),
- a step of determining (E2), for at least one identified carrier (P), at least one primary identifier (NUM_A),
- a step of searching (E3) in the proximity base (DB1) of all the time-stamped geographic positions (GD(A)) for said primary identifier (NUM_A) over a predetermined contagion time period (T_CONT),
- a step of determining (E4), in the proximity base (DB1), secondary identifiers (NUM_B(A)) which have secondary time-stamped geographic positions (GD(B)) that are common with the primary time-stamped geographic positions (GD(A)), so as to determine a base of potential carriers (DB_PP) of the virus, the determination step (E4) comprising:
∘ a sub-step of classifying (E41) the primary geographic positions (G(A)) of each primary individual (A) according to time ranges (TF) of a predetermined duration,
∘ a sub-step of determining (E42), for each time range (TF), a contagion region (RC) in which said primary geographic positions (G(A)) are included,
∘ a sub-step of determining (E43), in the proximity base (DB1), secondary identifiers (NUM_B(RC)) which have secondary time-stamped geographic positions (GD(B)) which belong temporally to said time range (TF) and geographically to the contagion region (RC),
∘ a sub-step of aggregating contagion regions (RC) obtained over a global time range (TFG) corresponding to a plurality of consecutive time ranges (TF),
∘ a sub-step of determining, in the proximity base (DB1), secondary identifiers (NUM_B(RC)) which have secondary time-stamped geographic positions (GD(B)) which belong temporally to said global time range (TFG) and geographically to the global contagion region (RCG),
∘ a sub-step of determining the duration of appearance of the secondary identifiers (NUM_B(RC)) in said global time range (TFG),
∘ a sub-step of preserving the secondary identifiers (NUM_B(RC)) whose duration of appearance is greater than a first predetermined time threshold (SE1),
∘ a sub-step of determining at least one overlapping zone (ZR) of contagion regions (RC) obtained over a global time range (TFG) corresponding to a plurality of consecutive time ranges (TF),
∘ a sub-step of determining, in the proximity base (DB1), secondary identifiers (NUM_B(RC)) which have secondary time-stamped geographic positions (GD(B)) which belong temporally to said global time range (TFG) and geographically to the overlapping zone (ZR),
∘ a sub-step of determining the duration of appearance of the secondary identifiers (NUM_B(RC)) in said global time range (TFG) and
∘ a sub-step of preserving the secondary identifiers (NUM_B(RC)) whose duration of appearance is greater than a second predetermined time threshold (SE2),
∘ a step of contacting (E5) the individuals associated with the secondary identifiers (NUM_B(A)) of the base of potential carriers (DB_PP).

2. The management method according to claim 1, wherein the first time threshold (SE1) is greater than the second time threshold (SE2).

3. A computer program for carrying out a method according to any of claims 1 to 2.

4. A computer storage medium comprising the computer program according to claim 3.
